# FASCICULE DE BREVET EUROPEEN

(11) **EP 1 394 145 B1**
(45) Date de publication et mention de la délivrance du brevet: **29.03.2006**
(21) Numéro de dépôt: 03026475.8
(22) Date de dépôt: 17.03.2000
(51) Int. Cl.: C07C 68/02, C07C 69/96

(54) **Procédé de préparation des fluoroformiates aliphatiques**
Verfahren zur Herstellung aliphatischer Fluoroformiate
Process for the production of aliphatic fluoroformates

(30) Priorité: 02.04.1999 FR 9904125
(43) Date de publication de la demande: 03.03.2004
(62) Demande divisionnaire de: 00910982.8
(73) Titulaire: ISOCHEM, 75194 Paris Cedex 04 (FR)
(72) Inventeur: Delabrouille, Philippe, 91150 Brouy (FR); Grenouillat, Denis, 91200 Athis Mons (FR); Senet, Jean-Pierre, 77760 La Chapelle La Reine (FR); Sennyey, Gérard, 91190 Gif Sur Yvette (FR)
(74) Mandataire: Ahner, Francis

(56) Documents cités:
- ALDRICH P E ET AL: "Alpha-fluorinated Ethers. II. Alkyl Fluoroalkyl Ethers" JOURNAL OF ORGANIC CHEMISTRY, AMERICAN CHEMICAL SOCIETY. EASTON, US, vol. 29, no. 1, 13 janvier 1964 (1964-01-13), pages 11-15, XP002140047 ISSN: 0022-3263
- JOHN CUOMO ET AL.: "An Efficient and Convenient Synthesis of Fluoroformates and Carbamoyl Fluorides" JOURNAL OF ORGANIC CHEMISTRY., vol. 44, no. 6, 16 mars 1979 (1979-03-16), pages 1016-1017, XP002134146 AMERICAN CHEMICAL SOCIETY. EASTON., US ISSN: 0022-3263

## Description

La présente invention concerne un procédé de préparation des fluoroformiates aliphatiques par réaction des alcools avec le fluorure de carbonyle. Elle concerne en particulier la préparation des fluoroformiates au moyen du fluorure de carbonyle obtenu à partir du phosgène.

Les fluoroformiates sont des composés connus, utiles comme produits intermédiaires notamment pour former des fluorures d'alkyle. Certains sont particulièrement utiles pour protéger le groupe amino des amino-acides.

Les fluoroformiates peuvent être préparés, par échange d'halogène, à partir des chloroformiates analogues, en les faisant réagir avec du fluorure de potassium. Cette méthode ne peut cependant pas être utilisée lorsque les composés sont instables ou possèdent des fonctions ou carbones réactifs dans la molécule.

Plusieurs autres procédés de préparation des fluoroformiates ont été proposés mais ils ne donnent pas entière satisfaction. Selon un des plus anciens procédés, décrit dans le brevet français n° 1 549 815, la préparation du fluoroformiate de t-butyle est effectuée en faisant réagir le fluorochlorure ou le fluorobromure de carbonyle avec le tertiobutanol mais ce procédé présente plusieurs inconvénients. Le fluorochlorure et le fluorobromure de carbonyle sont très difficiles à préparer et par conséquent très peu courants. La température au début de la réaction doit être très basse, aux environs de - 70°C et un cycle complexe de température de - 70°C à 0°C doit ensuite être mis en oeuvre, ce qui entraîne des frais opératoires très élevés. Le fluoroformiate obtenu est impur en raison des sous-produits formés ou du produit de départ non transformé.

Selon un autre procédé, la réaction de l'alcool est effectuée avec un mélange de phosgène et de phosgènes fluorés, en présence d'isobutylène et sous des pressions élevées, comme décrit dans le brevet FR n° 2 010 922, mais alors des installations particulières sont nécessaires.

Selon le brevet européen n° 176 412, on prépare les fluoroformiates en faisant réagir un carbonate alpha-chloré avec un fluorure alcalin mais la préparation du carbonate de départ nécessite une matière première supplémentaire et plusieurs étapes. De plus, la réaction du carbonate avec le fluorure produit le fluoroformiate avec un aldéhyde qu'il faut éliminer.

Des essais au laboratoire de préparation de fluoroformiates, à partir du phosgène, ont été effectués. Il a été mélangé à une température de - 78°C, du phosgène avec du fluorure de sodium, dans un solvant comprenant en majorité du sulfolane puis les produits résultants ont été mis à réagir avec du fluorure de potassium et l'alcool, mais les résultats obtenus n'ont pas pu être reproduits.

Il existait, par conséquent, un besoin d'un procédé de préparation des fluoroformiates aliphatiques qui soit simple, reproductible et qui permette d'obtenir les fluoroformiates avec de bons rendements et une bonne stabilité. On a maintenant découvert un procédé qui présente ces caractéristiques.

Selon le procédé de l'invention, on fait réagir, du fluorure de carbonyle avec un alcool aliphatique, en présence du fluorure de sodium qui est sous forme de poudre dont les grains ont une surface spécifique supérieure ou égale à 0.1 m²/g, dans un solvant choisi parmi les éthers, à une température comprise entre environ - 20° et environ 50°C.

Le terme aliphatique doit être compris comme couvrant les radicaux saturés ou non, substitués ou non, aliphatiques, cycloaliphatiques et araliphatiques.

Le procédé convient particulièrement bien pour préparer les fluoroformiates de tertiobutyle, de benzyle, d'adamantyle, de fluorénylméthyle, de tertioamyle ou d'allyle.

Les rendements en fluoroformiates obtenus grâce à ce procédé sont excellents. Le taux de conversion peut avoisiner les 100%.

La quantité de fluorure de carbonyle utilisée par rapport à l'alcool est de préférence de 1,1 à 2 moles par mole de l'alcool et plus particulièrement de 1,1 à 1,5 mole par mole.

La réaction du fluorure de carbonyle avec l'alcool s'effectue de préférence en présence d'une quantité voisine de la stoechiométrie et mieux d'un excès de fluorure de sodium. En particulier, on utilise une quantité de 1,1 à 2 moles du fluorure de sodium par mole de l'alcool, et de façon encore plus préférée supérieure à 1,15 mole par mole de l'alcool.

On a de plus trouvé qu'il est préférable que les grains de fluorure de sodium ont un diamètre moyen inférieur ou égal à 20 µm. De préférence, les grains ont une surface spécifique supérieure ou égale à 0,1 m²/g et de façon encore plus préférée également un diamètre moyen inférieur ou égal à 20 µm.

On a également trouvé qu'il est préférable de faire réagir progressivement le fluorure de carbonyle avec l'alcool et aussi de l'ajouter dans le milieu réactionnel qui contient l'alcool. Contrairement à ce qu'on pouvait attendre, le carbonate symétrique, sous-produit de la réaction, ne se forme pas, ce qui est surprenant dans la mesure où au début de la réaction il y a un défaut de fluorure de carbonyle par rapport à l'alcool.

Les éthers qui sont utilisés comme solvant dans la réaction du fluorure de carbonyle avec l'alcool sont cycliques ou non et sont par exemple, l'éther de tertiobutyle et de méthyle, le dioxanne, le tétrahydrofuranne, le 2-méthyltétrahydrofuranne, l'éther de dibenzyle, les diméthyléthers d'éthylèneglycol ou de polyéthylèneglycol (glymes). Le diméthoxyéthane et le diméthyléther du tétraéthylèneglycol en particulier conviennent bien.

La quantité de solvant pour cette réaction est généralement de 1 à 3 litres de solvant par kilogramme de fluoroformiate à obtenir.

La température de la réaction est quand à elle de préférence comprise entre environ - 5° et 40°C.

Il est préférable d'effectuer la réaction avec des composés anhydres et dans des conditions anhydres.

On a de plus trouvé que contrairement aux indications de l'art antérieur, il est important, pour obtenir les meilleurs résultats, et notamment d'excellents rendements, que le fluorure de carbonyle soit d'une très grande pureté et en particulier pratiquement exempt de composés chlorés tel qu'en particulier de phosgène et de fluorochlorure de carbonyle (COFCl).

En particulier, le fluorure de carbonyle de très grande pureté est obtenu en faisant réagir du phosgène, du diphosgène ou du triphosgène, ou un de leurs mélanges, avec un excès de fluorure de sodium en poudre dont les grains ont une surface spécifique supérieure ou égale à 0,1 m²/g et/ou un diamètre moyen inférieur ou égal à 20 µm, dans un solvant choisi parmi les solvants polaires et aprotiques, à une température comprise entre environ 25°C et environ 120°C, puis en faisant passer les gaz présents dans un condenseur dont la température est comprise entre environ 0° et environ - 50°C.

En réalisant le procédé de préparation du fluorure de carbonyle avec l'ensemble de ces conditions, on obtient à la sortie du condenseur, du fluorure de carbonyle avec une pureté très élevée, ne contenant pas de fluorochlorure de carbonyle et pratiquement pas de phosgène.

L'absence de ces deux gaz est particulièrement intéressante car on évite ainsi la formation de chloroformiates comme sous-produits, ce qui entraînait auparavant une diminution des rendements obtenus en fluoroformiates. De plus, les chloroformiates sont des composés très instables et on évite ainsi les risques de décomposition violente.

Les caractéristiques de la poudre de fluorure de sodium sont importantes pour une bonne réalisation de ce procédé. On a en effet constaté que lorsque les grains de fluorure de sodium ne présentent pas les caractéristiques décrites précédemment, la pureté du fluorure de carbonyle est nettement moins élevée et les rendements en fluorure de carbonyle et en fluoroformiates sont nettement plus faibles.

De préférence, les grains de fluorure de sodium ont une surface spécifique supérieure à 0,1 m²/g et de façon encore plus préférée également un diamètre moyen inférieur à 20 µm.

La poudre de fluorure de sodium doit être en excès par rapport au phosgène. De préférence, on utilise une quantité de 3 à 5 moles de fluorure de sodium par mole de phosgène.

Le solvant, bien sûr inerte vis à vis des réactifs, est choisi parmi les solvants qui sont aprotiques et polaires, c'est à dire les solvants dont la constante diélectrique est supérieure à 10 et de préférence supérieure à 20. Les nitriles aliphatiques conviennent bien. De préférence, on utilise l'acétonitrile.

La température du milieu réactionnel est de préférence comprise entre environ 35° et 80°C. La température du condenseur est quant à elle en particulier comprise entre environ - 20°C et - 40°C.

Le phosgène et/ou ses précurseurs sont de préférence introduits progressivement, dans le milieu réactionnel. Le phosgène est en général utilisé sous forme gazeuse. Il peut aussi être introduit sous forme de solution dans le solvant.

Le diphosgène ou le triphosgène sont introduits, en général en phase liquide, éventuellement en solution_dans le solvant, en quantités suffisantes pour donner la quantité de phosgène souhaitée.

La réaction est de préférence réalisée avec des composés et dans des conditions anhydres.

Le fluorure de carbonyle obtenu à la sortie du condenseur ne contient pas de fluorochlorure de carbonyle. Il contient d'infimes quantités de phosgène. Sa pureté déterminée par chromatographie en phase gazeuse est le plus souvent supérieure à 99% et son rendement est généralement supérieur à 95%.

Ce fluorure de carbonyle peut être directement utilisé pour préparer les fluoroformiates et de préférence il est mis à réagir au fur et à mesure de sa formation. La réaction du phosgène avec le fluorure de sodium est alors effectuée dans un premier réacteur, à une température de préférence comprise entre environ 35°C et 80°C. On utilise une quantité de phosgène au moins stoechiométrique par rapport à l'alcool que l'on souhaite transformer, et de préférence de 1,1 à 2 moles de phosgène par mole de l'alcool.

La quantité de fluorure de sodium que l'on fait réagir avec le phosgène est dans ce cas de préférence de 3 à 6 moles par mole de l'alcool à transformer et la quantité de solvant pour cette première réaction est généralement de 0,3 à 0,6 litre par mole de l'alcool.

Les gaz qui se dégagent du milieu réactionnel passent à travers le condenseur et sont introduits au fur et à mesure dans la solution de l'alcool contenue dans le deuxième réacteur.

La température du condenseur est de préférence comprise entre environ - 20°C et - 40°C. Les liquides condensés par le condenseur sont généralement recyclés dans le premier réacteur.

Le fluorure de sodium utilisé dans le deuxième réacteur est de préférence un fluorure de sodium ayant les mêmes caractéristiques que celui utilisé dans le premier réacteur.

Cette méthode préférée de préparation des fluoroformiates présente de grands avantages. Les manipulations sont réduites. Le procédé est plus économique et plus simple. Les rendements sont excellents et voisins de 100%.

Le procédé à partir du phosgène dure, en général, quelques heures. Lorsque la réaction est terminée, on sépare la solution de fluoroformiate du milieu réactionnel, généralement par filtration.

Pour obtenir le fluoroformiate encore plus pur, on peut le traiter avec un fluorure alcalin, de préférence avec du fluorure de sodium et en particulier de mêmes caractéristiques granulométriques que précédemment décrites. On effectue en général ce traitement avec le fluoroformiate en solution. On peut encore parfaire la purification en effectuant une distillation.

On a également trouvé un moyen d'obtenir les fluoroformiates qui sont solides à la température ambiante, généralement d'environ 20°C, très purs, sous forme cristallisée. Pour ce faire, on ajoute dans la solution du fluoroformiate, un composé qui ne dissout pas le fluoroformiate, choisi parmi les solvants aprotiques non polaires, en particulier de constante diélectrique inférieure à 10, et de préférence choisi parmi les alcanes, tel que le pentane, l'hexane, l'heptane et en particulier l'ISOPAR G ou l'ESSENCE G puis on refroidit la solution pour faire précipiter le fluoroformiate. Sa pureté déterminée par analyses est alors généralement supérieure à 99%.

Il peut être intéressant de conserver en solution, les fluoroformiates qui sont généralement instables. On a découvert que l'on améliorait de façon considérable la stabilité des fluoroformiates en solution, lorsqu'on ajoute à la solution que l'on veut conserver, environ 1 à 3 % en poids de diméthylformamide par rapport au fluoroformiate. On peut ainsi conserver cette solution plusieurs mois.

Le fluoroformiate en solution peut être utilisé directement pour effectuer d'autres réactions telle que, par exemple, la réaction avec des acides aminés.

Le procédé est illustré par les exemples qui suivent.

Sauf mention contraire, dans ces exemples, les réactions de préparation des fluoroformiates et du fluorure de carbonyle sont effectuées avec des composés, des appareillages et dans des conditions anhydres.

### EXEMPLE 1 : Préparation du fluoroformiate de tertiobutyle avec préparation du fluorure de carbonyle

Dans un premier réacteur, on a placé 189 g (4,5 moles) de fluorure de sodium en poudre dont les grains ont un diamètre moyen de 8,6 *µ*m et une surface spécifique de 0,27 m²/g et 340 ml d'acétonitrile. Ce premier réacteur est surmonté d'un condenseur maintenu à - 30°C qui est relié à un deuxième réacteur dans lequel on a placé 74 g (1 mole) de tertiobutanol et 49 g (1,17 mole) de fluorure de sodium de mêmes caractéristiques que précédemment et 150 ml de tétraglyme (diméthyléther du tétraéthylèneglycol), les deux réacteurs sont munis d'un système d'agitation. On chauffe le premier réacteur à une température de 50° C et la température du deuxième réacteur est maintenue aux environs de + 5°C. On introduit progressivement dans le milieu solvant, 148,5 g (1,5 mole) de phosgène gazeux pendant environ 4 heures. On analyse par chromatographie gazeuse et spectroscopie de masse, les gaz à la sortie du condenseur. On ne trouve pas trace de fluorochlorure de carbonyle et seulement des traces de phosgène en quantité inférieure à 0,1% en masse. La pureté du fluorure de carbonyle est supérieure à 99%. Le rendement déterminé par analyse des sels restants est de 98%.

L'obtention du fluoroformiate de tertiobutyle terminée, les gaz sont éliminés par un courant d'azote. Le contenu du deuxième réacteur est filtré et le gâteau est rincé avec quelques millilitres de tétraglyme.

Par analyse RMN ¹H, on constate que la conversion en fluoroformiate de tertiobutyle est de 100 %.

### EXEMPLE 2 : Préparation du fluoroformiate de tertiobutyle

Pour cet exemple, on utilise le fluorure de carbonyle le plus pur vendu dans des bouteilles acier et sous pression par la Société Union Carbide.

On relie cette bouteille à un réacteur de même type que le deuxième réacteur de l'exemple précédent qui contient les mêmes quantités de composés avec les mêmes caractéristiques et on opère dans les mêmes conditions. On introduit progressivement 1 mole de fluorure de carbonyle.

On constate que la conversion (déterminée par analyse RMN ¹H) en fluoroformiate de tertiobutyle est alors de 93%.

### EXEMPLE 3 : Préparation du fluoroformiate de tertiobutyle

Dans un premier réacteur, on a placé 30 g (0,7 mole) de fluorure de sodium dont les grains ont un diamètre moyen de 15 µm et une surface spécifique de 0,2 m²/g et 76 ml d'acétonitrile, et dans un deuxième réacteur, on a placé 11,1 g (0,15 mole) de tertiobutanol, 11 g (0,26 mole) de fluorure de sodium de mêmes caractéristiques que celui du premier réacteur et 25 ml de monoglyme (diméthoxyéthane) . Les deux réacteurs sont reliés comme précédemment par l'intermédiaire d'un condenseur à - 30°C. On chauffe le premier réacteur à une température de 55° à 60°C et on maintient le deuxième réacteur à une température de 20**°** à 25°C. On introduit dans le milieu réactionnel, 18,5 g (0,19 mole) de phosgène gazeux en trois heures. La réaction terminée, on fait passer un courant d'azote. On filtre le mélange réactionnel issu du deuxième réacteur sur une précouche de fluorure de sodium de mêmes caractéristiques. On rince le gâteau avec quelques millilitres de monoglyme. On recueille ainsi le fluoroformiate de tertiobutyle en solution dans le monoglyme. La quantité obtenue de ce fluoroformiate déterminée par analyse chromatographique en phase gazeuse est de 18 g soit un rendement de 100 %. On ajoute à cette solution, 0,36 g de diméthylformamide. La solution a pu être conservée 6 mois à une température comprise entre 0° et 5°C.

### EXEMPLE 4 : Préparation du fluoroformiate de tertiobutyle

Dans le premier réacteur, on a placé 75,6 g (1,8 mole) de fluorure de sodium dont les grains ont un diamètre moyen de 12 *µ*m et une surface spécifique de 0,23 m²/g et 100 ml d'acétonitrile. Dans le deuxième réacteur, on a placé 22,2 g (0,3 mole) de tertiobutanol, 14,7 g (0,35 mole) de fluorure de sodium identique à celui du premier réacteur et 40 ml de tétraglyme. On chauffe le premier réacteur à 80°C, on maintient à - 30°C la température du condenseur et à 5°C la température du deuxième réacteur. On introduit en moins d'une heure dans le premier réacteur, 44,6 g (0,15 mole) de triphosgène dans 100 ml d'acétonitrile. On laisse réagir pendant deux heures et on dose le fluoroformiate formé par RMN ¹H. La conversion en fluoroformiate de tertiobutyle est de 100 %.

Dans un autre essai, on a remplacé le triphosgène par une quantité équivalente de diphosgène. Les résultats obtenus sont identiques.

### EXEMPLE 5 : Préparation du fluoroformiate de benzyle

On opère comme à l'exemple 1, avec dans le premier réacteur 168 g (4 moles) de fluorure de sodium en poudre possédant les mêmes caractéristiques que décrites à l'exemple 1 et 320 ml d'acétonitrile et dans le deuxième réacteur, 108 g (1 mole) d'alcool benzylique, 50,5 g (1,2 mole) de fluorure de sodium de mêmes caractéristiques que précédemment et 150 g de diméthoxyéthane.

Après introduction de 120 g de phosgène, dégazage et filtration de la suspension contenue dans le second réacteur, on élimine le solvant par évaporation sous pression réduite puis on effectue une distillation fractionnée. On recueille ainsi 137 g de fluoroformiate de benzyle (rendement 89%), liquide incolore, dont les caractéristiques sont les suivantes :
Point d'ébullition : 64°C / 533 Pg (4 mm Hg),
RMN ¹H (CCl₄) δ : 7,42(s, 5H), 5,25 (s, 2H)

### EXEMPLE 6 : Préparation du fluoroformiate de 1-adamantyle

On opère comme à l'exemple précédent, le fluorure de sodium utilisé étant identique, mais avec, dans le premier réacteur 84 g (2 moles) de fluorure de sodium et 170 g d'acétonitrile et dans le deuxième réacteur 76 g (0,5 mole) de 1-adamantanol, 25 g (0,6 mole) de fluorure de sodium ainsi que 100 g de diméthoxyéthane.

Après introduction de 62 g de phosgène, dégazage et filtration de la suspension contenue dans le deuxième réacteur, on élimine le solvant par évaporation à 45°C sous 0,1 mm Hg. On recueille ainsi 90 g (rendement 91%) de fluoroformiate de 1-adamantyle, produit solide, avec les caractéristiques suivantes :
Point de fusion : 32°-33°C,
Spectre IR : 1830 cm⁻¹.

### EXEMPLE 7 : Préparation du fluoroformiate de 9-fluorénylméthyle (Fmoc-F)

On opère comme à l'exemple 1 mais avec du fluorure de sodium dont les grains ont un diamètre moyen de 9,5 µm et une surface spécifique de 0,25 m²/g.

Le premier réacteur contient 160 g (3,8 moles) de fluorure de sodium et 310 ml d'acétonitrile et le second réacteur 196 g (1 mole) de 9-fluorénylméthanol à 99,5% (HPLC), 50 g (1,19 mole) de fluorure de sodium et 340 g de diméthoxyéthane. Après introduction de 120 g de phosgène dans le premier réacteur, dégazage et filtration du contenu du second réacteur, on recueille environ 570 g d'une solution limpide de couleur marron clair. La conversion en Fmoc-F (déterminée par analyse RMN ¹H) est de 100%.

On ajoute à 200 g de cette solution chauffée à 50°C, 200 ml d'Isopar G également chauffés à 50°C et on concentre le tout jusqu'à 220 ml en maintenant la température toujours supérieure à 30° C. On filtre ensuite sur célite à une température toujours supérieure à 30°C, on rince le gâteau avec 50 ml d'essence G à une température supérieure à 30°C. On refroidit ensuite lentement le filtrat à 0°C, on filtre les cristaux obtenus, on rince deux fois avec de l'essence G à 0°C (100 ml et 50 ml). Après séchage à 20°-30°C, on obtient 58,5 g (rendement global 69%) d'un produit cristallisé blanc de point de fusion 41°C et de titre en Fmoc-F supérieur à 99% (déterminé par analyse HPLC).

### Exemple comparatif : Préparation du fluoroformiate de tertiobutyle

On opère comme à l'exemple 1, mais en utilisant un fluorure de sodium en poudre dont les grains ont une surface spécifique de 0,09 m²/g.

La conversion (déterminée par analyse RMN ¹H) en fluoroformiate de tertiobutyle est de 40% seulement.

## Revendications

1. Procédé de préparation d'un fluoroformiate aliphatique à partir d'un alcool aliphatique, **caractérisé en ce qu'**on fait réagir le fluorure de carbonyle avec l'alcool aliphatique, dans un solvant choisi parmi les éthers, à une température comprise entre - 20**°** et 50°C, en présence du fluorure de sodium qui est sous forme de poudre dont les grains ont une surface spécifique supérieure ou égale à 0,1 m²/g.

2. Procédé selon la revendication 1, **caractérisé en ce que** les grains de fluorure de sodium ont un diamètre moyen inférieur ou égal à 20 µm.

3. Procédé selon la revendication 1 ou 2, **caractérisé en ce que** le fluorure de carbonyle est introduit progressivement dans le milieu réactionnel qui contient l'alcool.

4. Procédé selon l'une quelconque des revendications précédentes, **caractérisé en ce que** la quantité de fluorure de carbonyle utilisée est de 1,1 à 2 moles par mole d'alcool.

5. Procédé selon l'une quelconque des revendications précédentes, **caractérisé en ce que** le fluorure de carbonyle est obtenu par réaction du phosgène, du diphosgène ou du triphosgène, ou d'un de leurs mélanges, avec un excès de fluorure de sodium en poudre dont les grains ont une surface spécifique supérieure ou égale à 0,1 m²/g et/ou un diamètre moyen inférieur ou égal à 20 µm, dans un solvant choisi parmi les solvants polaires et aprotiques, à une température comprise entre 25° et 120°C, et après passage des gaz présents, dans un condenseur dont la température est comprise entre 0° et - 50°C.

6. Procédé selon la revendication 5, **caractérisé en ce que** les grains de fluorure de sodium ont une surface spécifique supérieure ou égale à 0,1 m²/g.

7. Procédé selon la revendication 5 ou 6, **caractérisé en ce que** les grains de fluorure de sodium ont un diamètre moyen inférieur ou égal à 20 µm.

8. Procédé selon la revendication 5, 6 ou 7, **caractérisé en ce que** l'on fait réagir le phosgène avec le fluorure de sodium.

9. Procédé selon la revendication 8, **caractérisé en ce que** la quantité de fluorure de sodium mis à réagir avec le phosgène est de 3 à 5 moles par mole de phosgène.

10. Procédé selon l'une quelconque des revendications 5 à 9, **caractérisé en ce que** le phosgène et/ou ses précurseurs sont introduits progressivement.

11. Procédé selon l'une quelconque des revendications 5 à 10, **caractérisé en ce que** le solvant est l'acétonitrile.

12. Procédé selon l'une quelconque des revendications 5 à 11, **caractérisé en ce que** les liquides condensés par le condenseur sont recyclés dans le milieu réactionnel.

13. Procédé selon l'une quelconque des revendications précédentes, **caractérisé en ce que** la quantité de fluorure de sodium utilisée lors de la réaction de l'alcool avec le fluorure de carbonyle est comprise entre 1,1 et 2 moles par mole de l'alcool.

14. Procédé selon l'une quelconque des revendications précédentes, **caractérisé en ce que** pour la réaction de l'alcool avec le fluorure de carbonyle, le solvant est choisi parmi l'éther de tertiobutyle et de méthyle, le dioxanne, le tétrahydrofuranne, le 2-méthyltétrahydrofuranne, l'éther de dibenzyle, les diméthyléthers d'éthylèneglycol ou de polyéthylèneglycol.

15. Procédé selon l'une quelconque des revendications précédentes, **caractérisé en ce qu'**on purifie le fluoroformiate obtenu en le traitant avec un fluorure alcalin.

16. Procédé selon l'une quelconque des revendications précédentes, **caractérisé en ce qu'**on ajoute dans la solution du fluoroformiate, 1 à 3% en poids de diméthylformamide.

17. Procédé selon l'une quelconque des revendications précédentes, **caractérisé en ce qu'**on obtient le fluoroformiate lorsqu'il est un solide, sous forme cristallisée, en ajoutant dans la solution du fluoroformiate, un composé ne dissolvant pas le fluoroformiate choisi parmi les solvants aprotiques non polaires, puis on fait précipiter le fluoroformiate.

18. Procédé selon l'une quelconque des revendications précédentes, **caractérisé en ce que** l'on opère avec des composés et dans des conditions anhydres.

19. Procédé selon l'une quelconque des revendications précédentes, **caractérisé en ce que** l'alcool aliphatique est choisi dans le groupe qui comprend le tertiobutanol, l'alcool benzylique, l'adamentanol, le fluorényl-méthanol, l'alcool tertioamylique et l'alcool allylique.

## Patentansprüche

1. Verfahren zur Herstellung eines aliphatischen Fluorformiats ausgehend von einem aliphatischen Alkohol, **dadurch gekennzeichnet, dass** man Carbonylfluorid mit dem aliphatischen Alkohol reagieren lässt in einem Lösemittel, welches unter den Ethern ausgewählt wird, bei einer Temperatur zwischen -20° und 50°C eingeschlossen in Gegenwart von Natriumfluorid, welches in Form von Pulver, dessen Körner eine spezifische Oberfläche über oder gleich 0,1 m²/g aufweisen, vorliegt.

2. Verfahren nach Anspruch 1, **dadurch gekennzeichnet, dass** die Natriumfluoridkörner einen mittleren Durchmesser unter oder gleich 20 µm aufweisen.

3. Verfahren nach Anspruch 1 oder 2, **dadurch gekennzeichnet, dass** das Carbonylfluorid dem Reaktionsmedium, welches den Alkohol enthält, nach und nach zugesetzt wird.

4. Verfahren nach einem der vorangegangenen Ansprüche, **dadurch gekennzeichnet, dass** die eingesetzte Menge von Carbonylfluorid 1,1 bis 2 Mole pro Mol Alkohol beträgt.

5. Verfahren nach einem der vorangegangenen Ansprüche, **dadurch gekennzeichnet, dass** das Carbonylfluorid erhalten wird durch Umsetzung von Phosgen, von Diphosgen oder von Triphosgen oder von einer von deren Mischungen mit einem Überschuss von Natriumfluorid in Form von Pulver, dessen Kömer eine spezifische Oberfläche über oder gleich 0,1 m²/g und/oder einen mittleren Durchmesser unter oder gleich 20 µm aufweisen, in einem Lösemittel, welches unter den polaren und aprotischen Lösemitteln ausgewählt wird, bei einer Temperatur zwischen 25° und 120°C eingeschlossen und nach Durchleiten der vorhandenen Gase durch einen Kondensator, dessen Temperatur zwischen 0° und -50°C eingeschlossen liegt.

6. Verfahren nach Anspruch 5, **dadurch gekennzeichnet, dass** die Natriumfluoridkörner eine spezifische Oberfläche über oder gleich 0,1 m²/g aufweisen.

7. Verfahren nach Anspruch 5 oder 6, **dadurch gekennzeichnet, dass** die Natriumfluoridkörner einen mittleren Durchmesser unter oder gleich 20 µm aufweisen.

8. Verfahren nach Anspruch 5, 6 oder 7, **dadurch gekennzeichnet, dass** man Phosgen mit dem Natriumfluorid reagieren lässt.

9. Verfahren nach Anspruch 8, **dadurch gekennzeichnet, dass** die Menge von Natriumfluorid, welche man mit dem Phosgen reagieren lässt, 3 bis 5 Mole pro Mol Phosgen beträgt.

10. Verfahren nach einem der Ansprüche 5 bis 9, **dadurch gekennzeichnet, dass** das Phosgen und/oder dessen Vorstufen nach und nach eingespeist werden.

11. Verfahren nach einem der Ansprüche 5 bis 10, **dadurch gekennzeichnet, dass** das Lösemittel Acetonitril ist.

12. Verfahren nach einem der Ansprüche 5 bis 11, **dadurch gekennzeichnet, dass** die durch den Kondensator kondensierten Flüssigkeiten in das Reaktionsmedium zurückgeführt werden.

13. Verfahren nach einem der vorangegangenen Ansprüche, **dadurch gekennzeichnet, dass** die Menge von Natriumfluorid, welche während der Umsetzung des Alkohols mit dem Carbonylfluorid eingesetzt wird, zwischen 1,1 und 2 Molen pro Mol des Alkohols eingeschlossen liegt.

14. Verfahren nach einem der vorangegangenen Ansprüche, **dadurch gekennzeichnet, dass** für die Umsetzung des Alkohols mit dem Carbonylfluorid das Lösemittel ausgewählt wird unter tert.-Butylmethylether, Dioxan, Tetrahydrofuran, 2-Methyltetrahydrofuran, Dibenzylether, den Dimethylethem von Ethylenglycol oder von Polyethylenglycol.

15. Verfahren nach einem der vorangegangenen Ansprüche, **dadurch gekennzeichnet, dass** man das erhaltene Fluorformiat reinigt, indem man dieses mit einem Alkalimetallfluorid behandelt.

16. Verfahren nach einem der vorangegangenen Ansprüche, **dadurch gekennzeichnet, dass** man der Lösung des Fluorformiats 1 bis 3 Gew.% Dimethylformamid zusetzt.

17. Verfahren nach einem der vorangegangenen Ansprüche, **dadurch gekennzeichnet, dass** man das Fluorformiat gewinnt, wenn es ein Feststoff ist, in kristallisierter Form, indem man der Lösung des Fluorformiats eine Verbindung zusetzt, die das Fluorformiat nicht löst, welche ausgewählt wird unter den aprotischen, nicht-polaren Lösemitteln, und man dann das Fluorformiat präzipitieren lässt.

18. Verfahren nach einem der vorangegangenen Ansprüche, **dadurch gekennzeichnet, dass** man mit Verbindungen und unter Bedingungen, welche wasserfrei sind, arbeitet.

19. Verfahren nach einem der vorangegangenen Ansprüche, **dadurch gekennzeichnet, dass** der aliphatische Alkohol ausgewählt wird aus der Gruppe, welche tert.-Butanol, Benzylalkohol, Adamentanol, Fluorenylmethanol, tert.-Amylalkohol und Allylalkohol umfasst.

## Claims

1. Process for preparing an aliphatic fluoroformate from an aliphatic alcohol, **characterized in that** carbonyl fluoride is reacted with the aliphatic alcohol, in a solvent chosen from ethers, at a temperature of between -20°C and 50°C, in the presence of sodium fluoride that is in the form of a powder whose grains have a specific surface of greater than or equal to 0.1 m²/g.

2. Process according to Claim 1, **characterized in that** the grains of sodium fluoride have an average diameter of less than or equal to 20 *µ*m.

3. Process according to Claim 1 or 2, **characterized in that** the carbonyl fluoride is introduced gradually into the reaction medium which contains the alcohol.

4. Process according to any one of the preceding claims, **characterized in that** the amount of carbonyl fluoride used is from 1.1 to 2 mol per mole of alcohol.

5. Process according to any one of the preceding claims, **characterized in that** the carbonyl fluoride is obtained by reacting phosgene, diphosgene or triphosgene, or a mixture thereof, with an excess of sodium fluoride powder whose grains have a specific surface of greater than or equal to 0.1 m²/g and/or an average diameter of less than or equal to 20 *µ*m, in a solvent chosen from polar aprotic solvents, at a temperature of between 25°C and 120°C, and after passage of the gases present into a condenser whose temperature is between 0°C and -50°C.

6. Process according to Claim 5, **characterized in that** the grains of sodium fluoride have a specific surface of greater than or equal to 0.1 m²/g.

7. Process according to Claim 5 or 6, **characterized in that** the grains of sodium fluoride have an average diameter of less than or equal to 20 µm.

8. Process according to Claim 5, 6 or 7, **characterized in that** phosgene is reacted with sodium fluoride.

9. Process according to Claim 8, **characterized in that** the amount of sodium fluoride reacted with the phosgene is from 3 to 5 mol per mole of phosgene.

10. Process according to any one of Claims 5 to 9, **characterized in that** the phosgene and/or its precursors are introduced gradually.

11. Process according to any one of Claims 5 to 10, **characterized in that** the solvent is acetonitrile.

12. Process according to any one of Claims 5 to 11, **characterized in that** the liquids condensed by the condenser are recycled into the reaction medium.

13. Process according to any one of the preceding claims, **characterized in that** the amount of sodium fluoride used during the reaction of the alcohol with carbonyl fluoride is between 1.1 and 2 mol per mole of the alcohol.

14. Process according to any one of the preceding claims, **characterized in that** for the reaction of the alcohol with carbonyl fluoride, the solvent is chosen from tert-butyl methyl ether, dioxane, tetrahydrofuran, 2-methyltetrahydrofuran, dibenzyl ether, ethylene glycol dimethyl ethers or polyethylene glycol dimethyl ethers.

15. Process according to any one of the preceding claims, **characterized in that** the fluoroformate obtained is purified by treating it with an alkaline fluoride.

16. Process according to any one of the preceding claims, **characterized in that** 1 to 3% by weight of dimethylformamide is added to the fluoroformate solution.

17. Process according to any one of the preceding claims, **characterized in that**, when it is a solid, the fluoroformate is obtained in crystalline form by adding to the fluoroformate solution a compound which does not dissolve the fluoroformate, chosen from non-polar aprotic solvents, after which the fluoroformate is made to precipitate.

18. Process according to any one of the preceding claims, **characterized in that** it is performed with anhydrous compounds and under anhydrous conditions.

19. Process according to any one of the preceding claims, **characterized in that** the aliphatic alcohol is chosen from the group comprising tert-butanol, benzyl alcohol, adamantanol, fluorenylmethanol, tert-amyl alchohol and allyl alcohol.
